(19) Europäisches Patentamt
European Patent Office
Office européen des brevets

(11) Publication number: **0 066 205**
**B1**

(12) # EUROPEAN PATENT SPECIFICATION

(45) Date of publication of patent specification: **04.09.85**

(51) Int. Cl.[4]: **C 07 C 65/11**, C 07 C 51/15

(21) Application number: **82104427.8**

(22) Date of filing: **19.05.82**

(54) **Process for preparation of 2-hydroxynaphthalene-3-carboxylic acid.**

(30) Priority: **28.05.81 JP 80073/81**

(43) Date of publication of application:
**08.12.82 Bulletin 82/49**

(45) Publication of the grant of the patent:
**04.09.85 Bulletin 85/36**

(84) Designated Contracting States:
**BE CH DE FR GB IT LI NL**

(56) References cited:
**DE-A-2 837 053**
**DE-A-3 048 706**

**Patent Abstracts of Japan, Vol. 4, No. 69, 22 May 1980, page 79C11**

(73) Proprietor: **Kabushiki Kaisha Ueno Seiyaku Oyo Kenkyujo**
**2-31, Koraibashi**
**Higashi-ku Osaka (JP)**

(72) Inventor: **Ueno, Ryuzo**
**10-27, Nango-cho**
**Nishinomiya-shi Hyogo-ken (JP)**
Inventor: **Sakota, Kazuyuki**
**1798-7, Kobayashi Sumiyoshi-cho**
**Higashinada-ku Kobe-shi Hyogo-ken (JP)**
Inventor: **Nakamura, Yasunori**
**7-14, Takatsuka-cho**
**Nishinomiya-shi Hyogo-ken (JP)**

(74) Representative: **Dr. E. Wiegand Dipl.-Ing. W. Niemann Dr. M. Kohler Dipl.-Ing. J. Glaeser Dr. H.-R. Kressin Patentanwälte**
**Herzog-Wilhelm-Strasse 16**
**D-8000 München 2 (DE)**

**EP 0 066 205 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing 2-hydroxynaphthalene-3-carboxylic acid by the reaction of potassium β-naphtholate with carbon dioxide.

2-Hydroxynaphthalene-3-carboxylic acid is useful as an intermediate for pigments and dyes. Industrially, it is produced by the reaction of sodium β-naphtholate with carbon dioxide, and a solid-gas phase reaction called the Kolbe-Schmitt reaction has been used traditionally. This method involves repetitions of carboxylation and the recovery of by-product β-naphthol under reduced pressure, and has the defect that a long reaction time of more than 30 hours is required, losses of β-naphthol are large because of the thermal non-uniformity of the reaction at high temperatures, changes in phase during the reaction make it difficult to control the reaction and thus to obtain stable yields, and the yields of 2-hydroxynaphthalene-3-carboxylic acid based on sodium β-naphtholate are no more than 36% by weight (Ullmanns Encyclopädie der technischen Chemie, vol. 12, München (1960) p. 606—609). In an attempt to remove this defect, there have been proposed a method which comprises carboxylating an alkali metal β-naphtholate under a carbon dioxide pressure of 29.43 to 127.53 bar (gauge) (30 to 130 kg/cm$^2$) using β-naphthol as a solvent (U.S. Patent No. 4,057,576), a method which comprises carboxylating a mixture of sodium β-naphtholate and potassium β-naphtholate (U.S. Patent No. 4,032,568), etc. These methods, however, have not gained commercial acceptance because they require the use of expensive devices, or the yield of the desired product is not satisfactory.

Recently, a method which comprises continuously reacting a mixture being liquid under the reaction conditions and consisting of alkali β-naphtholate, especially sodium β-naphtholate, β-naphthol and a light oil or kerosene in a specified ratio came into commercial operation, and a marked improvement in the yield and quality of the final product has been achieved. However, the yield of the final product based on sodium β-naphtholate in this method does not exceed 50% (U.S. Patent No. 4,239,913 and DE—A1—2 837 053).

Furthermore, Patent Abstracts of Japan, Volume 4, No. 69 (1980) page 79 C 11 describes the process for the preparation of 2-hydroxynaphthalene-3-carboxylic acid by reacting a liquid mixture of 1 mole of sodium β-naphtholate and 0.3 to 3 moles of β-naphthol, and an aromatic hydrocarbon and/or an aromatic ether in 0.2 to 5 times the amount by weight of sodium β-naphtholate with carbon dioxide at ≧180°C under $CO_2$ pressure of ≦14.715 bar (gauge) (15 kg/cm$^2$). The yields obtainable thereby are also not satisfying.

It is an object of this invention therefore to improve the industrial mass-production method of producing 2-hydroxynaphthalene-3-carboxylic acid, and especially to improve the yields of product.

In order to achieve this object, we studied a method by which the reaction of a mixture of potassium β-naphtholate and β-naphthol with carbon dioxide is carried out in the liquid phase. This study has led to the discovery that by using a specified liquid as a reaction medium, the amounts of β-naphthol (becomes freed during the addition or the reaction) distributed to the potassium β-naphtholate layer and the reaction medium layer can be conveniently controlled so that the yield of 2-hydroxynaphthalene-3-carboxylic acid can be increased greatly. Furthermore it has been found that by the use of a specific carbon dioxide pressure the yield can be surprisingly increased.

Thus, according to this invention, there is provided a process for preparing 2-hydroxynaphthalene-3-carboxylic acid, which comprises reacting a mixture being liquid under the reaction conditions and consisting of (1) an alkali β-naphtholate, (2) β-naphthol and (3) a reaction medium with carbon dioxide at a reaction temperature of at least 180°C, which is characterized by the fact that as alkali β-naphtholate there is used potassium β-naphtholate, the reaction medium is selected from the group consisting of aliphatic hydrocarbons, alicyclic hydrocarbons, aromatic hydrocarbons and aromatic ethers, and that a carbon dioxide pressure of at least 14.715 bar (gauge) (15 kg/cm$^2$) is used.

The process of this invention gives 2-hydroxynaphthalene-3-carboxylic acid in a high yield and purity by simple and economical means. The yield of the desired product based on potassium β-naphtholate reaches 80% or more. The by-product 2-hydroxynaphthalene-6-carboxylic acid can be easily separated, and utilized as a material for polymers and dyes. Work-up of the reaction mixture is simple and gives the desired 2-hydroxynaphthalene-3-carboxylic acid in high purity. Furthermore, β-naphthol added or formed as a by-product can be recovered almost completely. These favorable results are unexpected, and the present invention provides a marked improvement in the method of preparing 2-hydroxynaphthalene-3-carboxylic acid.

Examples of the aliphatic, alicyclic and aromatic hydrocarbons used as the reaction medium in this invention include gasoline, kerosene, light oils (gas oils), lubricant oils, white oils, mono-, di- or poly-alkylbenzenes, mono-, di- or poly-alkylnaphthalenes, diphenyl, alkyl-substituted diphenyls, diphenylalkanes, alkyl-substituted diphenylalkanes and hydrogenated triphenyls. Examples of the aromatic ethers are diphenyl ether, anisole and ditolyl ether. Petroleum hydrocarbons are preferred as the hydrocarbons, and light oils (gas oils), kerosene and mono-, di- or poly-alkylnaphthalenes are especially preferred reaction media. The boiling range of the reaction medium is preferably 150 to 400°C, especially 180 to 380°C.

The mixture of potassium β-naphtholate and β-naphthol need to be thoroughly dehydrated. The mixture which is liquid under the reaction conditions can be obtained by dehydrating potassium β-naphtholate and β-naphthol in the presence of the reaction medium, or dehydrating them in the absence of the reaction medium and then adding the reaction medium. Alternatively, it may be obtained by adding

potassium hydroxide in an amount less than the theoretical amount to β-naphthol and dehydrating them in the presence or absence of the reaction medium. Preferably, 0.01 to 5 moles, especially 0.03 to 3 moles, of β-naphthol is used per mole of potassium β-naphtholate. Free β-naphthol performs the action of liquefying the reaction system, the action of forming monopotassium 2-hydroxynaphthalene-3-carboxylate and potassium β-naphtholate from dipotassium 2-hydroxynaphthalene-3-carboxylate as schematically shown below

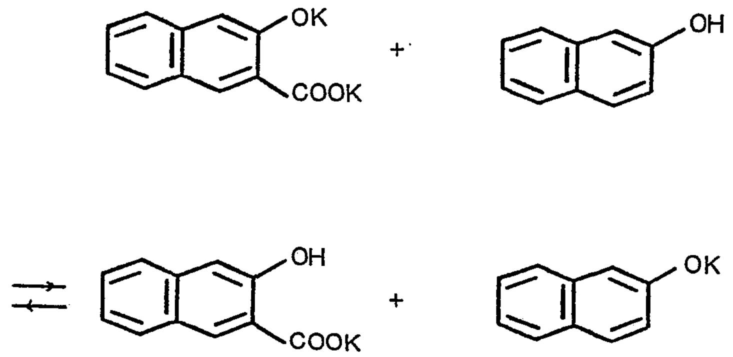

the action of increasing the yield of 2-hydroxynaphthalene-3-carboxylic acid by the carboxylation of potassium β-naphtholate, and the action of decomposing the by-product 2-hydroxynaphthalene-6-carboxylic acid salt.

The reaction of the aforesaid liquid mixture with carbon dioxide is carried out in accordance with this invention at a temperature of at least 180°C, preferably 230 to 350°C, especially preferably 240 to 330°C under a carbon dioxide pressure of at least 14.715 bar (gauge) (15 kg/cm$^2$). There is no particular upper limit to the carbon dioxide pressure, but from an economical viewpoint, the reaction can be carried out with the desired result at a carbon dioxide pressure of not more than 196.2 bar (gauge) (200 kg/cm$^2$), usually not more than 98.1 bar (gauge) (100 kg/cm$^2$). It is necessary that the reaction medium and the mixture of potassium β-naphtholate and β-naphthol should be liquid under the reaction conditions. The amount of the reaction medium, therefore, can vary according to the reaction conditions, especially the reaction temperature and the pressure of carbon dioxide. Usually, it is at least 0.5 times the weight of potassium β-naphtholate, preferably 0.5 to 10 times, especially preferably 0.5 to 5 times, the weight of the latter. Larger amounts of the reaction medium may be used, but better results in regard to the yield of the desired product cannot be obtained correspondingly. Rather, it is economically disadvantageous, and not beneficial to the work-up of the reaction mixture. Accordingly, the amount of the reaction medium is preferably not more than 10 times the weight of the potassium β-naphtholate.

The reaction medium performs the action of forming the starting mixture which is liquid under the reaction conditions, the action of adjusting the concentration of β-naphthol in the reaction system and thus increasing the rate of the carboxylation reaction, and the action of inhibiting the increasing of the viscosity of the reaction system to be caused by monopotassium 2-hydroxynaphthalene-3-carboxylate.

In a preferred embodiment of the present invention, the work-up is carried out in the following manner. Water is added to the reaction mixture after the reaction to separate it into a reaction medium layer and an aqueous layer. The reaction medium layer is separated, and β-naphthol is extracted from the aqueous layer using a hydrophobic extracting solvent which is preferably liquid at 110°C or below. As necessary, prior to the extraction, a tarry layer containing β-naphthol and a resinous material is sedimented in liquid form, preferably at 80 to 100°C, and separated. Preferably, the separated tarry layer is washed with water, and the washing is added together with water to the reaction mixture after the reaction.

Examples of suitable extracting solvents include hydrocarbons such as toluene, xylene, hexane and cyclohexane; halogenated hydrocarbons such as chlorobenzene, dichloromethane, dichloroethane and chloroform; nitrated hydrocarbons such as nitrobenzene and nitromethane; ethers such as dibutyl ether and diphenyl ether; ketones such as cyclohexanone, diisobutyl ketone and acetophenone; and alcohols having at least 4 carbon atoms such as n-butyl alcohol, n-octyl alcohol and 2-ethylhexyl alcohol.

The extraction is carried out at a temperature of preferably 30 to 110°C, especially preferably 50 to 100°C, by using the extracting solvent in an amount preferably 0.3 to 2 times, especially preferably 0.5 to 1.5 times, the volume of the aqueous layer. Preferably, β-naphthol in the reaction medium layer is recycled as such. β-Naphthol in the extracting solvent layer left after the separation of the reaction medium layer and the tarry layer is preferably recovered as a potassium hydroxide solution. β-Naphthol in the extracting solvent layer without the separation of the tarry layer, or in the tarry layer is recovered by vacuum distillation, etc. The recovered aqueous potassium β-naphtholate solution and β-naphthol can be recycled for reuse to the starting material preparing step. This extracting operation also results in the removal of a trace of the reaction medium in the aqueous solution.

Then, in order to separate the desired product from the aqueous layer, the aqueous layer after the extraction is adjusted to pH 1—3, preferably 1.5—2.5, and precipitated with an acid, if required after adjusting its pH to 4 to 6 and collecting 2-hydroxynaphthalene-6-carboxylic acid by filtration. 2-Hydroxynaphthalene-3-carboxylic acid is obtained in a substantially pure form or as a mixture with 2-hydroxynaphthalene-6-carboxylic acid. When it is necessary to separate the mixture into the constituent acids, it may, for example, be washed or recrystallized by using an organic solvent or a water-containing organic solvent.

The process of this invention can be practised either batchwise or continuously. The reaction and the work-up in a preferred embodiment of this invention are specifically described below with reference to the accompanying drawing which is a diagram showing the steps of the process of this invention when it is practised continuously.

A mixture consisting of potassium β-naphtholate, β-naphtol and the reaction medium prepared in a reservoir 1 is sent to a reaction vessel 2 wherein it is reacted at the reaction temperature and carbon dioxide pressure described hereinabove. The residence time is preferably 2 to 10 hours. The reaction mixture from the reaction vessel 2 is preferably cooled by a heat exchanger 3. Then, it is mixed with water fed from W in a water mixing tank 4 with stirring. It is then separated into a reaction medium layer and an aqueous layer in a separating tank 5. β-Naphthol may be recovered from the reaction medium layer (upper layer) R by using a recovering device (not shown). The aqueous layer (lower layer) from the separating tank 5 is adjusted to pH 6.5—8 in a pH adjusting tank 6 by adding an acid from A, and sent to an extracting device 7 (which is preferably a centrifugal extracting device) where it is extracted with a hydrophobic extracting solvent supplied from S. β-Naphthol can be recovered from the extracting solvent layer withdrawn from E, by using a recovering device (not shown). The aqueous layer from the extracting device 7 is sent to an acid precipitation tank 8, and precipitated with an acid added from A. The precipitate is separated by a centrifugal separator 9. The crystals obtained in the centrifugal separator are substantially pure 2-hydroxynaphthalene-3-carboxylic acid or a mixture of it with 2-hydroxynaphthalene-6-carboxylic acid. The mixture is separated, as required, into the individual acids using an alcohol, etc.

Advantageously, the work-up of the reaction mixture is carried out at a substantially constant temperature preferably in the range of 80 to 100°C. For this purpose, an ordinary thermal insulating or heating device may be used.

The aqueous potassium β-naphtholate solution and β-naphthol recovered from the reaction medium layer, the extracting solvent layer and the tarry layer which is separated as necessary are recycled to the starting material preparing step. The reaction medium and extracting solvent which have been separated are recycled respectively to the dehydrating device (not shown) and the extracting device 7 for re-use.

According to the process of this invention, the yield of 2-hydroxynaphthalene-3-carboxylic acid is usually increased to at least 80% after a reaction period of 2 to 10 hours. The total yield of it and the by-product 2-hydroxynaphthalene-6-carboxylic acid reaches at least 85%. The ratio of the two acids can be properly adjusted, and the proportion of 2-hydroxynaphthalene-3-carboxylic acid formed can be increased by adjusting the amount of free β-naphthol to a relatively large value or the carbon dioxide pressure to a relatively high value. The total yield of the two acids based on the consumed β-naphthol exceeds 90%, and the ratio of β-naphthol recovered is over 95%.

The reaction media used in this invention are relatively inexpensive, and readily available. In the process of this invention, it is not necessary to distill off β-naphthol freed during the reaction, but it can be re-used by recovering it as described hereinabove. The process of this invention is very advantageous in regard to thermal economy because the reaction medium and the extracting solvent can be recycled for re-use without the need for heating and cooling steps such as fractional distillation. Moreover, since there is scarcely any loss of the reaction medium and the extracting solvent by deterioration or otherwise, the ratio of these materials recovered is more than 99.5%. The process of this invention is also advantageous in regard to thermal economy because the separation of the reaction medium layer, the extraction of the aqueous layer and the optional separation of the tarry layer after the reaction can be carried out at much the same temperature. The process of this invention is very advantageous industrially in that the entire steps for the production of 2-hydroxynaphthalene-3-carboxylic acid from β-naphthol can be continuously carried out.

The following non-limitative Examples illustrate the present invention more specifically.

Example 1

A pressure reactor was charged with 364 g of light oil (boiling range 200 to 310°C) and 182 g of potassium β-naphtholate, and with stirring, 130 g of β-naphthol was added. They were reacted for 8 hours at a reaction temperature of 260°C and a carbon dioxide pressure of 29.43 bar (gauge) (30 kg/cm$^2$).

The reaction mixture was added to 830 ml of water, and dissolved by heating at 100°C for 30 minutes. Then, at 85°C, the solution is separated into a light oil layer and an aqueous layer. The aqueous layer was adjusted to pH 6.8 with dilute sulfuric acid, and extracted with 500 ml of toluene at 80°C. The aqueous layer was adjusted to pH 2.0 with dilute sulfuric acid, and cooled to 40°C to give 151.9 g of crystals. The crystals contained 146.7 g of 2-hydroxynaphthalene-3-carboxylic acid and 5.2 g of 2-hydroxynaphthalene-6-carboxylic acid (the amounts of the two acids were measured by gas chromatography after they were esterified with diazomethane). When the crystals were fractionated with dilute methanol, the individual acids in pure form were obtained.

The yield of 2-hydroxynaphthalene-3-carboxylic acid based on potassium β-naphtholate was 78.0%, and the total yield of the two acids was 80.8%. β-Naphthol was recovered from the light oil layer and the

toluene layer (its total amount was 147.2 g). The total yield of the two acids based on the consumed β-naphthol was 92.0%, and the ratio of β-naphthol recovered was 93.6%.

Example 2

A pressure reactor was charged with 546 g of kerosene (boiling range 180 to 280°C) and 182 g of potassium β-naphtholate, and with stirring, 259 g of β-naphthol was added. They were reacted for 8 hours at a reaction temperature of 240°C and a carbon dioxide pressure of 44.145 bar (gauge) (45 kg/cm$^2$).

The reaction mixture was added to 830 ml of water, and dissolved by heating at 100°C for 3 minutes. The solution was separated at 85°C into a light oil layer and an aqueous layer. The aqueous layer was adjusted to pH 6.8 with dilute sulfuric acid, and then at the same temperature, the sedimented tarry layer was separated. The aqueous layer was extracted with 500 ml of methyl isobutyl ketone at 80°C. The aqueous layer after the extraction was then adjusted to pH 2.0 with dilute sulfuric acid, and cooled to 40°C to give 138.4 g of 2-hydroxynaphthalene-3-carboxylic acid and 2.8 g of 2-hydroxynaphthalene-6-carboxylic acid, and 285 g of β-naphthol was recovered. The yield of 2-hydroxynaphthalene-3-carboxylic acid based on potassium β-naphtholate was 73.6%, and the total yield of the two acids was 75.1%. The yield of the two acids based on the consumed β-naphthol was 91.5%, and the ratio of β-naphthol recovered was 96.6%.

Example 3

A pressure reactor was charged with 364 g of 1-phenyl-1-(2,3-dimethylphenyl)ethane (boiling range 292 to 306°C) and 182 g of potassium β-naphtholate, and with stirring, 130 g of β-naphthol was added. They were reacted for 8 hours at a reaction temperature of 250°C and a carbon dioxide pressure of 29.43 bar (gauge) (30 kg/cm$^2$).

The reaction mixture was worked up in the same way as in Example 2 to give 131.6 g of 2-hydroxynaphthalene-3-carboxylic acid and 7.2 g of 2-hydroxynaphthalene-6-carboxylic acid, and 157.2 g of β-naphthol was recovered. The yield of 2-hydroxynaphthalene-3-carboxylic acid based on potassium β-naphtholate was 70.0%, and the total yield of the two acids was 73.8%. The yield of the two acids based on the consumed β-naphthol was 91.3%, and the ratio of β-naphthol recovered was 94.0%.

Example 4

A pressure reactor was charged with 546 g of Dowtherm A (a mixture of 75% of diphenyl ether and 25% of diphenyl having a boiling point of 257°C) and 182 g of potassium β-naphtholate, and with stirring, 259 g of β-naphthol was added. They were reacted for 8 hours at a reaction temperature of 250°C and a carbon dioxide pressure of 19.62 bar (gauge) (20 kg/cm$^2$).

The reaction mixture was worked up in the same way as in Example 2 to give 131.8 g of 2-hydroxynaphthalene-3-carboxylic acid and 7.1 g of 2-hydroxynaphthalene-6-carboxylic acid, and 286.7 g of β-naphthol was recovered. The yield of 2-hydroxynaphthalene-3-carboxylic acid based on potassium β-naphtholate was 70.1%, and the total yield of the two acids was 73.9%. The yield of the two acids based on the consumed β-naphthol was 91.3%, and the ratio of β-naphthol recovered was 96.6%.

Example 5

A pressure reactor was charged with 364 g of β-isopropylnaphthalene and 182 g of potassium β-naphtholate, and with stirring, 130 g of β-naphthol was added. They were reacted for 8 hours at a reaction temperature of 260°C and a carbon dioxide pressure of 58.86 bar (gauge) (60 kg/cm$^2$).

The reaction mixture was worked up in the same way as in Example 2 to give 134.1 g of 2-hydroxynaphthalene-3-carboxylic acid and 7.1 g of 2-hydroxynaphthalene-6-carboxylic acid, and 155 g of β-naphthol was recovered. The yield of 2-hydroxynaphthalene-3-carboxylic acid based on potassium β-naphtholate was 71.3%, and the total yield of the two acids was 75.1%. The yield of the two acids based on the consumed β-naphthol was 91.1%, and the ratio of β-naphthol recovered was 93.6%.

Example 6

The reaction and work-up were carried out continuously by using an apparatus of the type shown in the accompanying drawing.

A liquid dispersed mixture consisting of 182 parts by weight of light oil, 91 parts by weight of potassium β-naphtholate and 65 parts by weight of β-naphthol was prepared in the reservoir 1. The mixture was sent to the reaction vessel 2 maintained at a carbon dioxide pressure of 29.43 bar (gauge) (30 kg/cm$^2$) at a rate of 338 kg per hour, and reacted at 260°C. The residence time was 6 hours. The reaction mixture which left the reaction vessel was cooled by the heat exchanger 3, and with stirring, mixed with 400 liters/hr of water in the water mixing tank 4. The temperature of the mixture was adjusted to 85°C, and it was sent to the separating tank 5 where it was separated at 85°C into a light oil layer and an aqueous layer. β-Naphthol was recovered from the upper light oil layer by using a recovering device (not shown). The lower aqueous layer was adjusted to pH 6.8 with dilute sulfuric acid in the pH adjusting tank 6, and sent to the centrifugal extracting device 7 where it was extracted at 85°C with 250 liters/hour of xylene. The xylene layer was sent to a recovering device (not shown) to recover β-naphthol. The aqueous layer which left the extracting device was sent to the acid precipitating tank 8, and precipitated by adjusting its pH to 2.0 with dilute

sulfuric acid. 75.9 kg of crystals containing 73.9 kg of 2-hydroxynaphthalene-3-carboxylic acid and 2.0 kg of 2-hydroxynaphthalene-6-carboxylic acid were obtained hourly.

The yield of 2-hydroxynaphthalene-3-carboxylic acid based on potassium β-naphtholate was 78.6%, and the total yield of the two acids was 80.7%. 73.6 kg of β-naphthol was recovered hourly. The yield of the two acids based on the consumed β-naphthol was 91.9%, and the ratio of β-naphthol recovered was 93.6%.

Example 7

A pressure reactor was charged with 364 g of light oil and 182 g of potassium β-naphtholate, and with stirring, 259 g of β-naphthol was added. They were reacted for 8 hours at a reaction temperature of 250°C and a carbon dioxide pressure of 44.145 bar (gauge) (45 kg/cm$^2$).

The reaction mixture was added to 830 ml of water, and dissolved by heating at 100°C for 30 minutes. The solution was then separated at 85°C into a light oil layer and an aqueous layer. The aqueous layer was adjusted to pH 6.8 with dilute sulfuric acid, and extracted at 80°C with 500 ml of toluene. The aqueous layer after the extraction was adjusted to pH 4.3 with dilute sulfuric acid to precipitate 2-hydroxynaphthalene-6-carboxylic acid which was separated by filtration. The residue was adjusted to pH 2.0 with dilute sulfuric acid and cooled to 40°C to give 160.9 g of 2-hydroxynaphthalene-3-carboxylic acid. 265 g of β-naphthol was recovered. The yield of 2-hydroxynaphthalene-3-carboxylic acid based on potassium β-naphtholate was 85.6%, and the total yield of the two acids was 88.9%. The yield of the two acids based on the consumed β-naphthol was 92.7%, and the ratio of β-naphthol recovered was 96.3%.

**Claims**

1. A process for preparing 2-hydroxynaphthalene-3-carboxylic acid, which comprises reacting a mixture being liquid under the reaction conditions and consisting of (1) an alkali β-naphtholate, (2) β-naphthol and (3) a reaction medium with carbon dioxide at a reaction temperature of at least 180°C, characterized by the fact that as alkali β-naphtholate there is used potassium β-naphtholate, the reaction medium is selected from the group consisting of aliphatic hydrocarbons, alicyclic hydrocarbons, aromatic hydrocarbons and aromatic ethers, and that a carbon dioxide pressure of at least 14.715 bar (gauge) (15 kg/cm$^2$) is used.

2. The process of claim 1, characterized by the fact that 0.01 to 5 moles of β-naphthol is used per mole of potassium β-naphthol.

3. The process of claim 1, characterized by the fact that the reaction medium has a boiling point in the range of 150 to 400°C.

4. The process of claim 1, characterized by the fact that the reaction medium is a petroleum hydrocarbon.

5. The process of claim 4, characterized by the fact that the petroleum hydrocarbon is a light oil or kerosene.

6. The process of claim 1, characterized by the fact that the aromatic hydrocarbons are mono, di- or poly-alkylnaphthalenes.

7. The process of claim 1, characterized by the fact that the reaction temperature is 230 to 350°C.

8. The process of claim 1, characterized by the fact that the amount of the reaction medium is 0.5 to 10 times the weight of the potassium β-naphtholate.

9. The process of claim 1, characterized by the fact that water is added to the reaction mixture after the reaction to thereby separate a layer of the reaction medium, as required a tarry layer is sedimented in liquid form from the aqueous layer and is separated, the aqueous layer is extracted with a hydrophobic extracting solvent which is liquid at 110°C or lower to separate β-naphthol, and the aqueous layer left after the extraction is precipitated by using an acid.

**Patentansprüche**

1. Verfahren zur Herstellung von 2-Hydroxynaphthalin-3-carbonsäure durch Umsetzung einer bei den Reaktionsbedingungen flüssigen Mischung, bestehend aus (1) einem Alkali-β-naphtholat, (2) β-Naphthol und (3) einem Reaktionsmedium, mit Kohlendioxyd bei einer Reaktionstemperatur von wenigstens 180°C, dadurch gekennzeichnet, daß man als Alkali-β-naphtholat Kalium-β-naphtholat verwendet, das Reaktionsmedium aus der Gruppe, bestehend aus aliphatischen Kohlenwasserstoffen, alicyclischen Kohlenwasserstoffen, aromatischen Kohlenwasserstoffen und aromatischen Äthern, auswählt, und daß man bei einem Kohlendioxyddruck von wenigstens 14,715 Bar (gauge) (15 km/cm$^2$) arbeitet.

2. Verfahren nach Anspruch 1, dadurch gekennzeichnet, daß man 0,01 bis 5 Mole β-Naphthol pro Mol Kalium-β-naphthol verwendet.

3. Verfahren nach Anspruch 1, dadurch gekennzeichnet, daß das Reaktionsmedium einen Siedepunkt im Breich von 150 bis 400°C aufweist.

4. Verfahren nach Anspruch 1, dadurch gekennzeichnet, daß man als Reaktionsmedium einen Petroleumkohlenwasserstoff verwendet.

5. Verfahren nach Anspruch 4, dadurch gekennzeichnet, daß man als Petroleumkohlenwasserstoff Leichtöl oder Kerosin verwendet.

6. Verfahren nach Anspruch 1, dadurch gekennzeichnet, daß man als aromatische Kohlenwasserstoffe Mono-, Di- oder Polyalkylnaphthaline verwendet.

7. Verfahren nach Anspruch 1, dadurch gekennzeichnet, daß die Reaktionstemperatur bei 230 bis 350°C liegt.

8. Verfahren nach Anspruch 1, dadurch gekennzeichnet, daß die Menge des Reaktionsmediums bei dem 0,5- bis 10-fachen des Gewichtes des Kalium-β-naphtholats liegt.

9. Verfahren nach Anspruch 1, dadurch gekennzeichnet, daß man nach der Umsetzung Wasser zu der Reaktionsmischung zusetzt, um eine Schicht von dem Reaktionsmedium abzutrennen, von der wäßrigen Schicht eine Teerschicht in flüssiger Form aussedimentiert und abtrennt, die wäßrige Schicht mit einem hydrophoben Extraktionslösungsmittel, das bei 110°C oder darunter flüssig ist, extrahiert, um β-Naphthol abzutrennen, und die nach der Extraktion zurückbleibende wäßrige Schicht mit Säure versetzt, um die Ausfällung zu bewirken.

**Revendications**

1. Procédé de préparation de l'acide 2-hydroxynaphtalène-3-carboxylique, qui comprend la réaction d'un mélange, liquide dans les conditions de réaction et constitué (1) d'un β-naphtolate de métal alcalin, (2) de β-naphtol et (3) d'un milieu réactionnel, avec de l'anhydride carbonique à une température de réaction d'au moins 180°C, caractérisé par le fait que le β-naphtolate de potassium est utilisé à titre du β-naphtolate de métal alcalin, que le milieu réactionnel est choisi dans le groupe consistant en les hydrocarbures aliphatiques, les hydrocarbures alicycliques, les hydrocarbures aromatiques et les éthers aromatiques, et qu'est employée une pression d'anhydride carbonique d'au moins 14,715 bars (au manomètre) (15 kg/$cm^2$).

2. Procédé selon la revendication 1, caractérisé par le fait qu'il est fait usage de 0,01 à 5 moles de β-naphtol par mole de β-naphtolate de potassium.

3. Procédé selon la revendication 1, caractérisé par le fait que le milieu réactionnel présente un point d'ébullition situé dans la plage de 150 à 400°C.

4. Procédé selon la revendication 1, caractérisé par le fait que le milieu réactionnel est un hydrocarbure de pétrole.

5. Procédé selon la revendication 4, caractérisé par le fait que l'hydrocarbure de pétrole est une huile légère ou le kérosène.

6. Procédé selon la revendication 1, caractérisé par le fait que les hydrocarbures aromatiques sont des mono-, di- ou poly-alkylnaphtalènes.

7. Procédé selon la revendication 1, caractérisé par le fait que la température de réaction est de 230 à 350°C.

8. Procédé selon la revendication 1, caractérisé par le fait que la quantité du milieu réactionnel est de 0,5 à 10 fois le poids du β-naphtolate de potassium.

9. Procédé selon la revendication 1, caractérisé par le fait que de l'eau est ajoutée au mélange réactionnel après la réaction, de manière à séparer une phase de milieu réactionnel, si nécessaire une phase goudronneuse est sédimentée sous forme liquide à partir de la phase aqueuse et est séparée, la phase aqueuse est extraite avec un solvant d'extraction hydrophobe qui est liquide à 110°C ou moins pour séparer le β-naphtol, et la phase aqueuse restant après l'extraction est mise à précipiter par utilisation d'un acide.

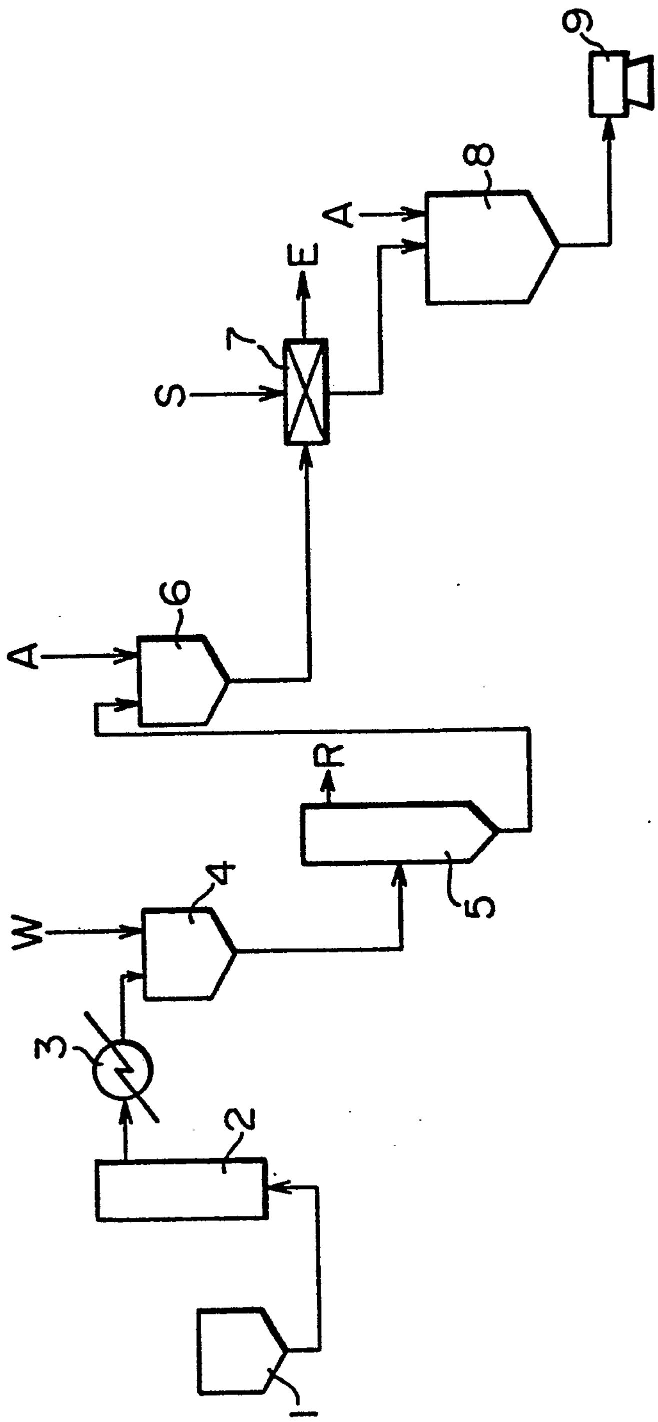
1
2
3
W
4
5
R
A
6
S
7
E
A
8
9